# EUROPEAN PATENT APPLICATION

(11) **EP 4 817 671 A1**
(43) Date of publication of application: **07.10.2026**
(21) Application number: 25168458.5
(22) Date of filing: 04.04.2025
(51) Int. Cl.: A61N 1/04, A61N 1/05, A61N 1/36

(54) **DEVICE FOR SELECTIVE VAGUS NERVE STIMULATION USING FOUR-ELECTRODE ADJUSTABLE CONFIGURATION**

(71) Applicant: UAB Pulsetto, 330096 Klaipeda (LT)
(72) Inventor: Sabaliauskas, Povilas, 10245 Vilnius (LT)
(74) Representative: Metida

(57) **Abstract**

The invention provides a neurostimulation device and method for selectively and precisely stimulating the vagus nerve. The device features a novel four-electrode configuration arranged in a square, enabling dynamic control of the electrical field. This configuration allows for accurate targeting of specific nerve fibers, such as A-beta and A-delta fibers, while avoiding the activation of undesired C fibers. The device includes an electrode distance adjustment mechanism, allowing the distance between electrodes to be varied from 20 mm to 90 mm, ensuring customization for individual patient anatomies. It also supports both bipolar and monopolar operation modes, offering flexibility in stimulation. The method involves dynamically adjusting the electrode configuration and electrical field to achieve precise, controlled stimulation tailored to the patient. This dynamic control enhances therapeutic efficacy, minimizes side effects, and offers an adaptable, effective solution for vagus nerve stimulation.

## Description

### FIELD OF THE INVENTION

The present invention is related to the field of neurostimulation devices and methods for non-invasive vagus nerve stimulation. Specifically, it addresses the use of a four-electrode configuration to generate a dynamically adjustable electrical field for selective nerve fiber stimulation. The invention provides a device and method for precisely targeting A- alfa (α) and A-beta (β) (motorized), A-delta (δ)(sensory) and B type fibers while avoiding activation of C fibers, improving therapeutic outcomes. The invention is set out in the appended set of claims.

### BACKGROUND

Non-invasive vagus nerve stimulation (nVNS) is a method of stimulating the vagus nerve without the need for surgery, using electrical impulses delivered through the skin, typically via electrodes placed on the neck. It has gained attention for its therapeutic potential in treating conditions like chronic pain, epilepsy, depression, and migraines, by modulating the brain's neurochemistry through vagus nerve stimulation. The non-invasive nature of nVNS makes it safer than traditional invasive treatments, with fewer risks such as infection or complications from surgery. It also allows for personalized treatment, with adjustable parameters tailored to individual patients, improving both efficacy and comfort. Additionally, nVNS can be used in outpatient settings, offering convenience for long-term management of various conditions. Overall, nVNS represents a promising, accessible, and effective alternative to more invasive therapies.

The object of the present invention is to provide a non-invasive vagus nerve stimulation device and method using a four-electrode configuration that allows for selective and precise stimulation of specific nerve fibers, such as Aα and Aβ, Aδ and B fibers, while avoiding activation of undesired C fibers. Traditional vagus nerve stimulators often rely on a two-electrode system, which may not allow for targeted stimulation or customization to individual patient anatomies.

A patent document EP2945690 describes a system for non-invasive vagus nerve stimulation aimed at treating medical conditions. The system includes a handheld stimulator with a contact surface for contacting the patient's skin, electrodes for generating an electric field, and a power source to drive the stimulation. The stimulator is coupled with a docking station that receives data from a remote source and transmits it to the stimulator, enabling the patient to receive therapy according to a specified regimen. The system allows the modulation of electric field amplitude and utilizes bursts of pulses with frequencies suitable for vagus nerve stimulation. However, this system lacks adjustability and precision.

Similarly, US20140142654 describes a system for non-invasive vagus nerve stimulation, which includes a stimulator with a housing that contacts the patient's skin and generates an electric current to modulate the nerve at a target region. The system also includes a docking station that communicates with external computers, receiving and transmitting data related to the therapy regimen. The stimulator is capable of modulating the amplitude of the electric field and performing various functions, including controlling the electric field parameters and receiving treatment instructions. However, this document does not provide customizability or selectivity in electrode positioning.

The closest prior art to the invention CN219185610U describes a dual-loop, crossed-electrode percutaneous vagus nerve stimulation system. It includes an electrode arrangement designed to generate a crossed electrical field, which provides broader and more stable coverage of the vagus nerve. This system features an adjustable setup for stimulating the nerve with various modes, such as synchronized frequency stimulation and burst stimulation. However, while this system offers flexibility and covers more extensive areas, it still faces issues such as limited precision in targeting specific nerve fibers and adapting to individual patient anatomies.

The prior art highlights several technical challenges in non-invasive vagus nerve stimulation, including the lack of precision in targeting specific nerve fibers like Aα and Aβ, Aδ and B fibers while avoiding C fiber. Existing devices typically use a two-electrode system with a fixed electrode distance, limiting the ability to precisely control the electrical field and adapt to individual patient anatomies. Additionally, many systems lack dynamic regulation of the electrode configuration, resulting in inconsistent stimulation coverage. These limitations emphasize the need for a device with adjustable electrode distances and more precise control over the electrical field for targeted and reliable nerve stimulation.

### SUMMARY OF THE DISCLOSURE

The invention concerns a non-invasive vagus nerve stimulation device and method that comprises a four-electrode configuration, configured to selectively and precisely stimulate specific nerve fibers, such as Aα and Aβ, Aδ and B fibers, while avoiding the activation of undesired C fibers. The device comprises an electrode distance adjustment mechanism, configured to adjust the distance between the electrodes from 20 mm to 90 mm, for customizing the device to individual patient anatomies, ensuring that the electrical field can be tailored for optimal stimulation. This dynamic regulation of the electrode configuration allows the electrical field to be controlled, ensuring optimal stimulation for enhanced effectiveness.

### BRIEF DESCRIPTION OF THE DRAWINGS

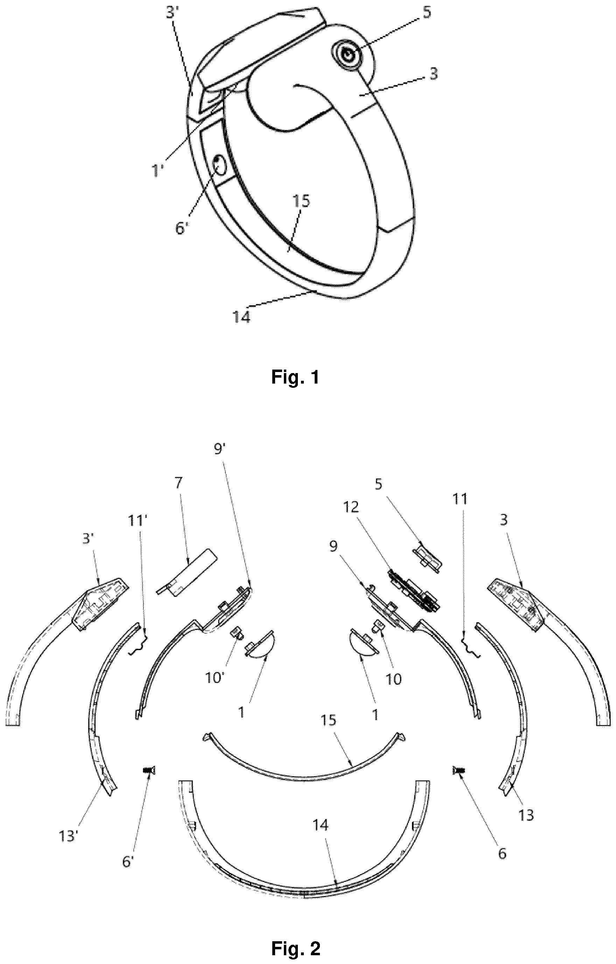

FIG. 1 is a perspective view of a stimulation device, with four-electrode configured to adjust the distance between electrodes according to an embodiment of the present invention;
Fig. 2 is an exploaded view of a stimulation device, with four-electrode configured to adjust the distance between electrodes according to an embodiment of the present invention;
Fig. 3 closer view of electrode placement according to an embodiment of the present invention;
Fig. 4 is a diagram view showing the electric field movement on both sides of the device in when distance between the electrodes 20 cm.
Fig. 5 is a diagram view showing the electric field movement on both sides of the device in when distance between the electrodes 90 cm.
Fig. 6 is a diagram view showing the electric field movement on both sides of the device in when distance between the electrodes 45 cm.

### List of reference signs:

1 - top electrode right
1' - top electrode left
2 - bottom electrode right
2' - bottom electrode left
3 - right unit
3' - left unit
4 - USB type port
5 - power button
6 - self tapping screw right
6' - self tapping screw left
7 - battery
8 - top right
8' - top left
9- bottom right
9' - bottom left
10 - M3 screw right
10' - M3 screw left
11 - spring right
11' - spring left
12 - PCB
13 - slider right
13' - slider left
14 - arch
15-arch lid

### DETAILED DESCRIPTION OF THE INVENTION

The present invention relates to a non-invasive vagus nerve stimulation device and method, which utilizes a four-electrode configuration for the precise and selective stimulation of specific nerve fibers. The device is configured to adjust the distance between electrodes, offering customization for individual patient anatomies, thereby ensuring the optimal electrical field for therapeutic stimulation. This invention provides a dynamic regulation of the electrical field, allowing for the targeting of Aα and Aβ, Aδ and B fibers, while avoiding the activation of undesired C fibers. The detailed description below outlines the technical components and functionality of the device, demonstrating its unique features and advantages over prior art systems.

Vagus nerve stimulator for targeting selective nerve fibers for stimulation comprising:
an electrode for generating the necessary electrical field to stimulate the vagus nerve;
a means for generating an electrical field;
characterized in that the device also comprises the following elements:
   a four-electrode configuration, arranged in a square for generating the necessary electrical field to stimulate the vagus nerve in a controlled and directional manner;
   an electrode distance adjustment mechanism to adjust the distance between the electrodes (from 20 mm to 90 mm) for customizing the device to individual patient anatomies, ensuring the electrical field can be tailored for optimal stimulation;
   a means for generating a dynamic electrical field, for selectively targeting specific nerve fibers (such as A-beta and A-delta fibers) and avoiding undesired activation of C fibers. The present invention relates to a non-invasive vagus nerve stimulator designed to selectively stimulate specific nerve fibers in the vagus nerve.

The device incorporates a configuration of at least four-electrodes (1, 1', 2, 2') made of biologically compatible material allowed to be used on the body, for example, 304 stainless steel. Top electrode right (1) (+) is placed on the right side of the device and serves as part of the four-electrode configuration. It helps generate the electrical field required for stimulating the vagus nerve, playing a critical role in directional control and selective targeting of specific nerve fibers. Tope electrode left (1') (+) is the counterpart to the top electrode right and is positioned on the left side of the device. Together with the top right electrode, it contributes to the four-electrode system, allowing for precise and adjustable electrical field generation for effective vagus nerve stimulation. Bottom electrode right (2) (-) is positioned on the right bottom of the device, this electrode works with the other electrodes to complete the electrical circuit, ensuring the accurate delivery of electrical impulses to the targeted area of the vagus nerve. It also aids in the directional control of the electrical field. Bottom electrode left (2') (-) is positioned on the left bottom of the device, complementing the bottom right electrode in creating the dynamic electrical field. The placement of this electrode helps optimize the stimulation of the vagus nerve.

This configuration of four-electrodes (1, 1', 2, 2'). arranged in a square formation, which enables the generation of a dynamically adjustable and controlled electrical field. This configuration allows the electrical field to be tailored to the specific anatomical needs of individual patients, ensuring more precise targeting of desired nerve fibers, such as Aα and Aβ, Aδ and B fibers, while avoiding the activation of undesired C fibers.

The device's design overcomes the limitations of traditional vagus nerve stimulators, which typically rely on a two-electrode system. In these systems, the lack of precision in electrode placement and the fixed distance between electrodes often result in non-selective stimulation, leading to ineffective treatments or unwanted side effects. By incorporating a four-electrode configuration (1, 1', 2, 2'), the invention enables dynamic control over the electrical field, making it possible to target specific nerve fibers with greater accuracy.

A right unit (3) the right unit serves as the housing and support structure for the right electrodes (1, 2). It also contains the necessary components for controlling and adjusting the electrical field, ensuring the device functions effectively and comfortably for the patient. Left unit (3') houses the left electrodes (1', 2'). It provides additional support for the electrical system and helps in adjusting the device to individual patient needs.

USB type port (4) allows for external connectivity, such as charging the device. Bluetooth is used to transfer data related to the device's settings and usage. It facilitates communication between the device and external systems for data monitoring and adjustments.

A power button (5) powers the device on and off. It is used to initiate and terminate the stimulation process and controls the basic operational functions of the device.

A self-tapping right (6) and left (6') screws are used to secure various components of the device in place. They are located on the right side and ensure the right unit's structural integrity, holding together the parts of the device. These screws are counterparts to the self-tapping screws, ensuring secure assembly and structural stability on the left side of the device.

A battery (7) powers the entire device. It supplies the necessary energy to generate the electrical field for vagus nerve stimulation and ensures long-term usage without requiring a constant external power source.

A top right (8) and top left (8') part refers to the upper right and left sections of the device, where certain components are housed. It serves as a physical structure that helps in the overall device assembly.

A part mirrors the top right (8) and houses components related to the device's upper side. It ensures the structural and electrical integrity of the device.

A bottom right (9) and left (9') sections of the device serves as the base for mounting the right and left electrodes (1, 2), (1', 2') and ensuring that the device fits comfortably on the patient's skin and ensures the device is stable on the patient's body during use for optimal and effective stimulation.

M3 screws right (10) and left (10') are M3 screws that are used to secure the device's internal components on the right and left side. They are part of the assembly mechanism, ensuring the electrode arrangement and battery compartment are securely held in place. They play a similar role in holding internal components, ensuring device stability and proper function.

A spring right (11) and a spring left (11') are for the adjustment of the device.

PCB (12), the printed circuit board (PCB) is the central component of the device that manages all electrical functions. It controls the generation of the electrical field, adjusts the electrode settings, and regulates power delivery.

A slider right (13) and slider left (13') are mechanisms on the right and left sides of the device that enables adjustment of electrode distances. It allows for the precise tuning of the electrical field and electrode placement and dynamic control of the electrical field, ensuring that the device can be personalized to each patient's needs.

An arch (14) is the framework that holds the electrodes and other parts of the device in place. It helps maintain the structural integrity of the device, ensuring that the electrodes are properly positioned for stimulation.

An arch lid (15) covers and protects the internal components housed within the arch. It ensures that the device is secure, protecting sensitive components from damage or contamination.

The electrode distance adjustment mechanism is designed to allow customization of the electrical field, optimizing nerve stimulation based on individual patient anatomy. By adjusting the electrode distance, the mechanism modifies the shape and intensity of the electrical field, ensuring effective and comfortable treatment.

The means for generating a dynamic electrical field comprises several additional components that works together to produce, regulate, and control the electrical field used for vagus nerve stimulation:
Printed circuit board (PCB) (12), configured to control electrical signal parameters, including frequency, pulse width, and intensity, manages real-time adjustments to the stimulation protocol to optimize nerve activation and avoid discomfort and configured to switch between bipolar and monopolar stimulation modes, adjusting how the electrical field is distributed between the electrodes; wherein bipolar mode: generates an electric field between adjacent electrodes for a localized stimulation and monopolar mode: expands the field by using one electrode as active, affecting a wider nerve area. Alternatively, it can be manually adjusted.
power supply system like battery (7), configured to provide the necessary electrical energy for generating the stimulation field, ensures stable and uninterrupted operation of the device, with control interface - the power button (5), configured to enable the user to adjust settings such as stimulation mode (bipolar/monopolar) and duration, allows manual or automated control over the dynamic electrical field. The stimulation intensity is controlled via the app, which also offers the option to use predefined parameters;
The USB type port (4) is configured for power supply.

Data transfer and firmware updates are handled via the Bluetooth protocol, facilitates real-time monitoring and adjustments of stimulation parameters.

The means for generating a dynamic electrical field ensures real-time adjustments of the field shape, intensity, and direction. This system allows personalized vagus nerve stimulation, improving treatment effectiveness while minimizing unwanted nerve activation or discomfort.

Here's how it works:
placing at least four-electrode configuration (1, 1', 2, 2') on a patient's skin in proximity to the vagus nerve;
adjusting the distance between the electrodes (1, 2) and (1', 2') using an electrode distance adjustment mechanism to customize the device to the patient's anatomy; generating an electrical field using a means for generating an electrical field; modulating the electrical field dynamically using a printed circuit board (12) to selectively stimulate specific nerve fibers, including Aα and Aβ, Aδ and B fibers, while avoiding activation of C fibers.

The device consists of four electrodes (1, 1', 2, 2') arranged in a square configuration. The distance between these electrodes adjusts to customize the electrical field and target specific nerve fibers more precisely, ensuring compatibility with variations in each patient's anatomical structure, particularly in the neck region where the vagus nerve is accessed.

An adaptable electrode distance mechanism consisting of sliders (13, 13') and springs (11, 11'),which allow for fine-tuning of the distance between the electrodes (1, 2) and (1', 2'). This mechanism controls the positioning of the electrodes, allowing fine-tuning based on the patient's needs. The mechanism operates through slides (13, 13'), which enable linear movement of the electrodes within a range of 20 mm to 90 mm. As the sliders move, they either bring the electrodes closer together or push them farther apart. This movement modifies the distance between the top right (1) and top left electrodes (1') and the bottom right (2) and bottom left electrodes (2'), thereby altering the electrical field generated by the device.

The strength of the electric field (E) depends on the voltage (V) and the distance between the electrodes (d), according to the formula: E = V/d.

The calculation shows that at a depth of 1,3-1,5 cm, the electric field is approximately 50 V/m, which is sufficient to activate Aα and Aβ, Aδ and B fibers.

Due to the square electrode configuration, the field can be directed more precisely compared to a conventional two-electrode system.

By combining bipolar and monopolar modes, the intensity and direction of the electric field can be adjusted.

Aα and Aβ, Aδ and B fibers - responsible for sensation and pain modulation. They are activated at an electric field strength of 50 V/m.

C fibers - associated with slow, unpleasant pain, are activated only at field strengths above 240 V/m.

This system ensures that their activation threshold is not reached, preventing unwanted stimulation.

A closed-loop system, allow for automatic optimization of electrode positioning based on the user's physiological data. The distance over the long term is monitored using a wearable device. The device can be controlled via Bluetooth. Stimulation intensity and modes can be adjusted.

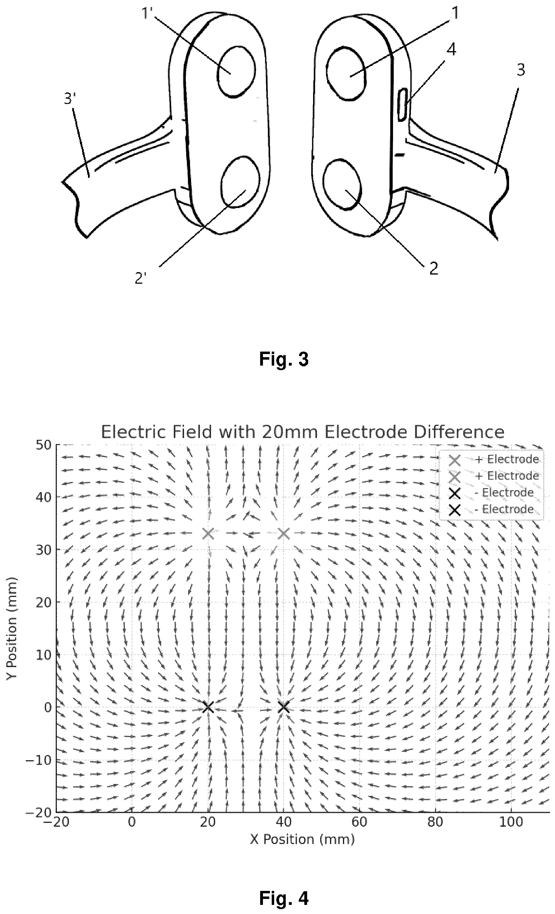

When the electrodes (1, 2) and (1', 2') move closer together, the electrical field becomes more concentrated and localized, enabling precise targeting of A-beta and A-delta fibers while avoiding the activation of C fibers. This configuration provides more intense stimulation for smaller areas. The image Fig. 4 illustrates the resulting electric field distribution when using a 20 mm electrode separation, showing how the arrows represent the electric field vector directions, indicating the flow and strength of the electrical forces applied to the nerve region. This visualization confirms how the four-electrode system enables precise control over the electrical stimulation area, allowing selective modulation for optimal vagus nerve activation.

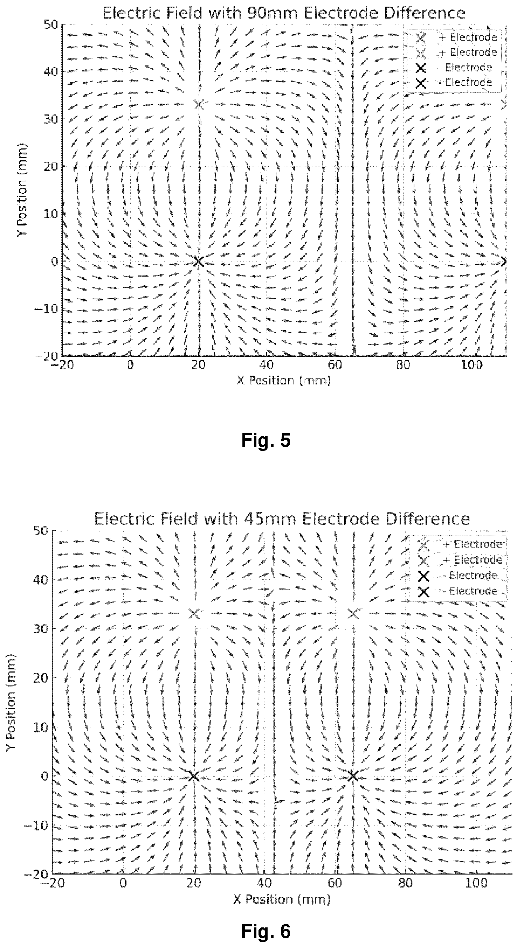

Conversely, when the electrodes move farther apart, the electrical field becomes more diffuse, covering a broader stimulation area. This setup benefits larger anatomical regions, where a widespread field ensures deeper or more extensive nerve stimulation. Fig. 6 illustrates the electric field distribution with a 45 mm electrode spacing, showing how the field shape changes with electrode distance. The positive electrodes (+ X) are positioned at the top left and top right, while the negative electrodes (- X) are at the bottom left and bottom right. The electric field vectors originate from the positive electrodes and curve toward the negative electrodes, forming a structured quadrupolar field. Compared to a 20 mm electrode difference, the wider spacing results in a broader and more dispersed field, covering a larger area while reducing localized intensity. This configuration enables precise nerve stimulation, allowing targeted activation of Aα and Aβ, Aδ and B fibers while minimizing undesired C fiber activation. The ability to adjust electrode distance helps in customizing the stimulation field based on individual anatomical needs, ensuring effective and comfortable vagus nerve stimulation.

Fig. 5 illustrates the electric field distribution with a 90 mm electrode spacing, demonstrating how an increased distance between electrodes affects field intensity and coverage. The positive electrodes (1) and (1') (+, light gray X) are placed at the top left and top right, while the negative electrodes (2) and (2') (-, black X) are at the bottom left and bottom right. The electric field vectors show a broader, more diffused pattern, resulting in a less concentrated but more extensive stimulation zone. Compared to smaller electrode distances (e.g., 20 mm or 45 mm), this configuration creates a weaker but more widespread field, which may be beneficial for targeting larger nerve areas while minimizing localized nerve overstimulation. The adjustable electrode mechanism allows fine-tuning of the field distribution to match individual anatomical requirements, ensuring an optimized balance between stimulation effectiveness and patient comfort.

The arch (14) and arch lid (15) secure the structural stability of the device, ensuring that the adjustable components remain in place during use. The self-tapping screws (6, 6') and M3 screws (10, 10') further support fixation of the device components, ensuring durability and preventing unintentional movement of the electrodes during operation. The power button (5) activates the device, while the USB-type port (4) allows for charging or external connectivity. The battery (7) supplies power to the printed circuit board (PCB) (12), which generates and regulates the electrical field. This combination serves as a means for generating an electrical field, ensuring stable and controlled neurostimulation.

The PCB controls the dynamic electrical field, enabling selective activation of specific nerve fibers and preventing undesired stimulation. Alternatively, it can be manually adjusted.

During operation, the device delivers electrical impulses to the skin at the stimulation site, typically the neck, via the four electrodes (1, 1', 2, 2'). Electrode gel or hydrogel can be used to reduce skin resistance like SignaGel but not limited to this gel alone. The generated electrical field interacts with the vagus nerve, producing neurostimulation. By adjusting electrode distance (3, 3') and electrical field parameters (regulated by PCB (12)), the device ensures targeted activation of the desired nerve fibers, improving therapeutic efficacy while minimizing side effects. The system incorporates software and hardware safety limits to ensure that the electric field does not exceed permissible thresholds.

The springs (11, 11') are designed for maintaining stability and allowing for precise adjustments. When looped shape of the device facilitates smooth and controlled movement of the sliders (13, 13') while maintaining stability.

The device's dynamic electrical field and adjustable electrode distance provide significant advantages over conventional vagus nerve stimulators. These features enhance precision, improve patient comfort, and enable personalized treatment for various medical conditions.

The method of use involves dynamically adjusting the electrode configuration (1, 1', 2, 2') and electrical field parameters in real-time according to the patient's individual requirements. This process ensures selective stimulation of specific nerve fibers, allowing controlled and precise modulation of the vagus nerve.

Overall, the invention provides a more effective, adaptable, and safe solution for non-invasive vagus nerve stimulation, offering a significant advancement over current technologies that rely on fixed electrode systems and lack precise control over the electrical field.

The present invention provides an advanced non-invasive vagus nerve stimulator that enhances the precision, adaptability, and effectiveness of neurostimulation therapy. Unlike traditional two-electrode stimulators, the device features a four-electrode configuration (1, 1', 2, 2') arranged in a square, allowing for dynamic and directional control of the electrical field. This innovation ensures selective stimulation of specific nerve fibers, such as Aα and Aβ, Aδ and B fibers, while avoiding the activation of unwanted C fibers.

A key feature of the invention is the adjustable electrode distance mechanism, which includes sliders (13, 13') and springs (11, 11'), enabling fine-tuned electrode positioning from 20 mm to 90 mm. This adjustability ensures personalization of stimulation based on individual patient anatomies, optimizing therapeutic efficacy and minimizing side effects. Additionally, the device supports both bipolar and monopolar stimulation modes, providing flexibility in electrical field generation. The incorporation of a power system (7, 12), control interface (5), and USB-type port (4) ensures ease of use, making the device suitable for both clinical and at-home applications.

By integrating precise electrode positioning, dynamic electrical field control, and an adjustable configuration, this invention addresses the limitations of existing vagus nerve stimulators. It offers a highly customizable, effective, and safe solution for treating a range of neurological and physiological conditions, representing a significant advancement in non-invasive neurostimulation technology. It can be used for neuromodulation in epilepsy, depression, and autonomic nervous system regulation, improving overall health and well-being.

## Claims

1. Vagus nerve stimulator for targeting selective nerve fibers for stimulation comprising:
an electrode for generating the necessary electrical field to stimulate the vagus nerve;
a means for generating an electrical field;
**characterized in that** the device also comprises the following elements:
at least four-electrode configuration, for generating the electrical field to stimulate the vagus nerve in a controlled and directional manner;
an electrode distance adjustment mechanism to adjust the distance between the electrodes for customizing the device to individual patient anatomies, ensuring the electrical field to be tailored for optimal stimulation;
a means for generating a dynamic electrical field, for selectively targeting specific nerve fibers Aα and Aβ, Aδ and B fibers) and avoiding undesired activation of C fibers.

2. The non-invasive vagus nerve stimulator according to claim 1, **characterized in that** the four-electrode configuration arranged in a square formation, including a top electrode right (1) and a bottom electrode right (2) arranged in a right unit (3), a top electrode left (1') and a bottom electrode left (2') arranged a left unit (3'), for generating a controlled and directional electrical field to stimulate the vagus nerve.

3. The non-invasive vagus nerve stimulator according to claim 2, **characterized in that** an electrode distance adjustment mechanism comprising the right unit (3) and the left unit (3'), (11, 11') and a slider mechanism (13, 13') and looped shape of the device enabling movement of the electrodes to customize the electrical field and modify the shape and strength of the electrical field dynamically for individual patient anatomies; that allows for adjustment of the distance between the electrodes within a range of 20 mm to 90 mm.

4. The non-invasive vagus nerve stimulator according to claim 1, **characterized in that** a means for generating a dynamic electrical field is a printed circuit board (12) configured to generate and regulate the electrical field, enabling selective stimulation of specific nerve fibers such as Aα and Aβ, Aδ and B fibers while avoiding activation of C fibers; a power supply battery (7) for providing the necessary energy to operate the device.

5. A method for selectively stimulating specific nerve fibers in a vagus nerve using a non-invasive vagus nerve stimulator, the method comprising:
placing at least four-electrode configuration (1, 1', 2, 2') on a patient's skin in proximity to the vagus nerve;
adjusting the distance between the electrodes (1, 2) and (1', 2') using an electrode distance adjustment mechanism to customize the device to the patient's anatomy;
generating an electrical field using a means for generating an electrical field;
modulating the electrical field dynamically using a printed circuit board (12) to selectively stimulate specific nerve fibers, including Aα and Aβ, Aδ and B fibers, while avoiding activation of C fibers.

6. The method according to claim 5, **characterized in that** it further comprises:
configuring the four-electrode configuration (1, 2) and (1', 2') in a square formation to generate a controlled and directional electrical field.

7. The method according to claim 5 or 6, **characterized in that** it further comprises:
adjusting the electrode distance within a range of 20 mm to 90 mm using sliders (13, 13') and spring elements (11, 11') to optimize electrical field distribution and ensure precise nerve fiber stimulation.

8. The method according to claim 5, **characterized in that** it further comprises:
adjusting stimulation parameters via an external interface manually or using Bluetooth communication with the app.
